Europäisches Patentamt

European Patent Office

Office européen des brevets

(19)

(11) Veröffentlichungsnummer : **0 113 094**
**B1**

(12) # EUROPÄISCHE PATENTSCHRIFT

(45) Veröffentlichungstag der Patentschrift :
**17.08.88**

(21) Anmeldenummer : **83112731.1**

(22) Anmeldetag : **17.12.83**

(51) Int. Cl.⁴ : **G 01 N 33/52**, G 01 N 33/50,
G 01 N 31/22

(54) Testvorrichtung und Verfahren zum Nachweis von Thiolen.

(30) Priorität : **24.12.82 GB 8236810**

(43) Veröffentlichungstag der Anmeldung :
**11.07.84 Patentblatt 84/28**

(45) Bekanntmachung des Hinweises auf die Patenterteilung : **17.08.88 Patentblatt 88/33**

(84) Benannte Vertragsstaaten :
**AT BE CH DE FR GB IT LI LU NL SE**

(56) Entgegenhaltungen :
**CH-A- 588 698**
**US-A- 3 864 085**
**US-A- 3 891 669**
**US-A- 3 891 670**
**US-A- 4 021 198**

(73) Patentinhaber : **Boehringer Ingelheim International G.m.b.H**
**D-6507 Ingelheim am Rhein (DE)**

(72) Erfinder : **Shaw, Ian Charles**
**International Hall University of London**
**Brunswick Square London, WC1 (GB)**
Erfinder : **Francis Dalton, Joan Isobel**
**25 St. Hubertus Close Gerrads Cross**
**Buckinghamshire (GB)**

EP 0 113 094 B1

**0 113 094**

## Beschreibung

Die vorliegende Erfindung betrifft eine Testvorrichtung, insbesondere eine Vorrichtung zum Nachweis von Thiolen in einer Körperflüssigkeit.

Weiterhin sind aus Beutler et al. J. Lab. and Clin. Med., 61, 882-888 (1963) und aus der US-A-386 4085 Testverfahren bekannt, mit denen man durch Reaktion mit 5′, 5′-Dithiobis-(2-nitrobenzoesäure) und anschließender Messung der optischen Dichte reduziertes Gluthathion im Blut bestimmen kann.

Nicht beschrieben sind Vorrichtungen, die es ohne Labor oder geschultes Laborpersonal erlauben, Thiole wie z. B. Natrium-2-mercaptoethansulfat (im folgenden MESNA genannt) Penicillamine, Cystein u.ä. in Körperflüssigkeiten direkt nachzuweisen.

MESNA wird bei der Krebstherapie mit Oxazaphosphorinen als Harnorgane schützendes Reagenz eingesetzt. Die Oxazaphosphorine (Cyclophosphamid, Ifosfamid und Trophosphamid) sind alkylierende Reagenzien, die vielfach bei der cytostatischen Therapie verwendet werden. Urotoxische Nebenwirkungen können ihre Anwendung einschränken. In der Blase können diese Nebenwirkungen von der erhöhten Miktionsfrequenz und Dysurie bis zur schweren hämorrhagischen Zystitis reichen und zu Fibrose, reduzierter Kapazität und sogar zu Blasentumoren führen.

Forcierte Diurese und Blasenspülungen werden routinemäßig durchgeführt um solche Komplikationen zu vermeiden. Das erfordert jedoch stationäre Behandlung und ist nicht nur zeitraubend, sondern unbequem und möglicherweise für den Patienten gefährlich.

MESNA, eine Sulfhydryl-Verbindung, kann die mit diesen Medikamenten einhergehende Urotoxizität verhindern.

Neben Vorrichtungen zum Nachweis oder zur Konzentrationsbestimmung verschiedener Substanzen in Körperflüssigkeiten, z. B. Tauchpapierstreifen zum Nachweis von Zucker im Urin ist aus CH-A-588 698 ein Teststreifen bekannt, mit dem Homocystin und Cystin in Körperflüssigkeiten nachgewiesen werden kann. Hierbei werden Homocystin und Cystin mit einem Reduktionsmittel reduziert und die erhaltenen Thiole mit dem Indikator Nitroprussidsalz nachgewiesen.

Es beeinträchtigt nicht den alkylierenden oder cytotoxischen Effekt der Oxazaphosphorine und reagiert auch nicht mit anderen cytotoxischen Verbindungen, wie BCNU, Doxorubicin, 5-FU, MTX, VCR oder Cisplatinum. MESNA wird in vivo schnell zum biologisch inaktiven Disulfid-DIMESNA oxidiert, das in der Niere wieder zur freien Thiolverbindung reduziert wird. MESNA reagiert im Urin mit Acrolein, das als Hauptmetabolit der Oxazaphosphorine für deren Urotoxizität verantwortlich ist, und bildet einen nichttoxischen stabilen Thioether, der dann unverändert ausgeschieden wird. Ebenso führt spontane Zersetzung des 4-Hydroxyoxazaphosphorins in der Blase zu Acrolein. MESNA vermindert die Zersetzungs-Rate der 4-Hydroxy-Metabolite der Oxazaphosphorine im Urin durch die Bildung eines relativ stabilen, nichturotoxischen Kondensationsproduktes und vermindert so die Rate freiwerdenden Acroleins.

Da die Halbwertszeiten der Metaboliten innerhalb des Organismus wesentlich größer sind als die von MESNA, und der Grad des Schutzes von der Konzentration an freiem Thiol im Urin abhängt, ist es von außerordentlicher Bedeutung, die Blasen-MESNA-Konzentration zu jeder Zeit auf einem, die Harnorgane schützenden Spiegel (veranschlagt auf einen Überschuß von annähernd 300 μg/ml) zu halten. Obwohl jedoch für normale Patienten solche MESNA-Dosen verabreicht werden, daß eine Konzentration von ca. 900 μg/ml Urin in der Blase erreicht wird, gibt es eine bedeutende Anzahl von Patienten, die bei normalen MESNA-Gaben geringere Konzentrationen in der Blase aufweisen und somit von den Metaboliten der alkylierenden Reagenzien gefährdet werden, wenn nicht der MESNA-Spiegel in der Blase beobachtet und erhöht wird.

Mögliche Gründe für die niedrigen MESNA-Spiegel in der Blase sind erhöhte Flüssigkeitsaufnahme, was zum Ausspülen von MESNA aus der Blase führt, und Nierenversagen.

Wenn geringe MESNA-Werte festgestellt werden, kann die MESNA-Dosierung so lange erhöht werden, bis ein, die Harnorgane schützender Spiegel erreicht ist.

Da jedoch große Überschüsse an MESNA möglicherweise zu unerwarteten Nebenwirkungen, wie Übelkeit führen, ist die Erhöhung der Dosierung nur dort angezeigt, wo normale Dosierungen sich als ungenügend herausgestellt haben.

Die Thiole, wie MESNA, in einer Urinprobe sind oxidationsempfindlich und wenn der Thiolgehalt der Probe nicht innerhalb 1/2 h nach der Sammlung festgestellt wird, erhält man ungenaue Ergebnisse. Um die vorhandenen Labormethoden, für die Thiolbestimmung nutzen zu können, muß die Oxidation chemisch unterbunden werden bevor die Proben zur Analyse gegeben werden.

Die Thiol-Bestimmung wird dann in Labor durch Zugabe von Ellman's Reagenz, eine Lösung, in der die aktive Substanz 5′, 5′-Dithio-bis(2-nitrobenzoesäure) (im folgenden « 5,5-DNB » genannt) ist, und durch Messung der Absorptionsänderung der Proben durchgeführt. In Gegenwart von Thiolen zeigt das Ellman's Reagenz eine tiefgelbe Farbe.

Die beschriebene Labormethode zur Bestimmung der Thiole im Urin erfordert hochentwickelte Laboreinrichtungen, ist zeitraubend und führt möglicherweise zu einer Verzögerung im Bereich von Stunden zwischen der Abgabe des Urins und der nötigen Änderung der Thioldosierung, die erfolgen muß, wenn ungenügende Thiolkonzentrationen festgestellt werden. Während dieser Verzögerung können die

2

oben beschriebenen, durch die Metaboliten der alkylierenden Reagenzien in der Blase hervorgerufenen gefährlichen und schmerzhaften Wirkungen eintreten. Um den MESNA-Spiegel der Blase zu überwachen und wenn nötig, anheben zu können, um diese Nebenwirkungen zu vermeiden, besteht ein Bedarf an einer neuen Methode zur Bestimmung von Thiolen in Körperflüssigkeiten wie Urin, die für eine schnelle Überwachung der Thiolspiegel geeignet ist.

Aufgabe der vorliegenden Erfindung ist es daher, eine Methode und eine Vorrichtung zum Nachweis von Thiolen in Körperflüssigkeiten zu entwickeln, die schnell und ohne Labor oder geschultes Laborpersonal eingesetzt werden kann.

Gegenstand der vorliegenden Erfindung ist eine Vorrichtung zum Nachweis von Thiolen in einer Körperflüssigkeit, dadurch gekennzeichnet, daß sie ein Absorptionsmaterial enthält, in dem der Indikator 5',5'-Dithiobis-(2-nitrobenzoesäure), ein Puffer und als Anti-Oxidans Flavonoide, Katechine, Ascorbinsäure, Chinole oder Chinolderivate absorbiert ist.

Ein weiterer Gegenstand der Erfindung ist eine Methode zum Nachweis von Thiolen in einer Körperflüssigkeit, dadurch gekennzeichnet, daß eine Vorrichtung, die ein Absorptionsmaterial enthält, in dem der Indikator 5',5'-Dithiobis-(2-nitrobenzoesäure), ein Puffer und als Anti-Oxidans Flavonoide, Katechine, Ascorbinsäure, Chinole oder Chinolderivate absorbiert ist, innerhalb einer halben Stunde nach der Entnahme in die zu testende Körperflüssigkeit eingetaucht wird und der Grad einer auftretenden Farbveränderung im allgemeinen 5 bis 10 Sekunden nach dem Kontakt der Vorrichtung mit der Probe, als semiquantitatives Maß für die Anwesenheit von Thiolen genommen wird.

Die erfindungsgemäße Vorrichtung enthält ein Anti-Oxidans, da bei dessen Abwesenheit festgestellt wurde, daß sich das Absorptionsmittel, das üblicherweise Papier, wie z. B. Filterpapier ist, verfärbt und dadurch den Nachweis der Farbveränderung ungenau werden läßt. Das Anti-Oxidans darf keine Thiolgruppen enthalten. Geeignete Substanzen sind Flavonoide und Katechine, Ascorbinsäure, Chinole und Chinolderivate.

Besonders bevorzugt ist Chinol, da es seine anti-oxidative Wirkung ohne Beeinträchtigung des farbverändernden Reagenzes zeigt. Geeignete Chinol-Konzentrationen im Absorptionsmittel, angenommen es ist Filterpapier, erhält man durch Eintauchen des Substrates in eine Lösung, die wenigstens 3,5 g/l, vorzugsweise wenigstens 4 g/l Chinol enthält. Als ein Reagenz, das in Gegenwart von Thiolen, wie MESNA, Penicillamin, Cystein o.ä. Farbveränderungen zeigt, eignet sich die aktive Komponente aus dem Ellmans's Reagenz. Bei Verwendung dieses Reagenzes 5,5-DNB verändert sich die Farbe des Absorptionsmittels unmittelbar nach Kontakt mit einer Urin-Probe, die genügende Konzentrationen an Thiol aufweist, nach leuchtendgelb.

Durch Veränderung der Reagenzkonzentration im Absorptionsmittel kann die Empfindlichkeit der Vorrichtung ebenso variiert werden, um sicherzustellen, daß der Farbwechsel für das spezielle Thiol, z. B. MESNA, erst oberhalb einer bestimmten akzeptablen Minimalkonzentration beobachtet wird. So kann, wenn beispielsweise das Absorptionsmaterial Whatman No. 1 Filterpapier in eine Lösung, die unter anderem 2 g/l 5,5-DNB enthält, eingetaucht und getrocknet wird, der Farbwechsel zum tiefgelben hin bei einer Urinprobe, die ungefähr 400 μg/ml oder mehr MESNA enthält, eintreten. Dadurch kann der normale Thiolspiegel im Urin eines Patienten, der keine Thiolbehandlung erhält und kleiner ist als ca. 50 μg/ml, mit der erfindungsgemäßen Vorrichtung kein positives Ergebnis bewirken.

Das Absorptionsmittel der erfindungsgemäßen Vorrichtung enthält einen Puffer, der sicherstellt, daß der pH für die Thiol/Reagenz-Reaktion den für den Farbwechsel optimalen Wert besitzt.

Der Puffer-pH hängt ab von der Wahl des Reagenzes ; für 5,5-DNB ist ein schwach alkalischer pH, üblicherweise ca. 7.4, angebracht. Nach einer vorteilhaften Ausführung der Erfindung hat die Vorrichtung die Form eines Kunststoffstreifens, dessen eines Ende das Absorptionsmittel trägt. Bei dieser Ausführungsform kommen die Finger des Ausführenden nicht mit der Probe oder dem Absorptionsmittel in Kontakt. Elution kann dazu führen, daß die Farbe auf dem Absorptionsmittel über die Oberfläche wandert. Zur Minimierung dieses Effektes kann das Absorptionsmittel, das vorzugsweise die Form einer Scheibe, eines Streifens oder eines Blattes bis zu einer Dicke von ca. 0.5 mm besitzt, an seinen großen Flächen von einem Plastikfilm überzogen werden, der nur die Ränder freiläßt.

Testvorrichtungen dieser Art können auf übliche Weise verpackt werden, z. B. in Flaschen über Silica Gel, das das Absorptionsmittel davor bewahrt, feucht zu werden.

Die erfindungsgemäße Vorrichtung und Methode ist schnell, nicht-invasiv und kann von Schwestern, dem Patienten oder Verwandten bei stationärer oder ambulanter Behandlung eingesetzt werden. Es ist eine einfache Möglichkeit, um den Thiolspiegel des Urins zu beobachten, und die es erlaubt, die MESNA-Dosierung unverzüglich anzupassen, um dadurch die urotoxischen Auswirkungen der Zeiten mit geringem MESNA-Spiegel im Urin zu verhindern.

MESNA besitzt einen breiten therapeutischen Index und kann intravenös oder oral gegeben werden. Die Anwendung des erfindungsgemäßen Teststreifens erlaubt die Anpassung der MESNA-Dosierung und sorgt dadurch für den Schutz gegen Urothel-Toxizität der Oxazaphosphorine. Bei ambulanter Oxazaphosphorin-Behandlung kann MESNA erfolgreich oral verabreicht werden. Veränderung der freien Thiolkonzentration im Urin kann mittels erfindungsgemäßem Teststreifen beobachtet werden, wodurch jede notwendige Modifikation der MESNA-Dosierung möglich wird.

Daher hat die Teststreifenmethode klinische Bedeutung für die stationäre und ambulante cytotoxische Oxazaphosphorin-Therapie. Dadurch, daß sie eine Anpassung der MESNA-Dosierung ermöglicht, wird nicht nur die Therapie effektiver, sondern genauso nachhaltig wird die Lebensqualität des Patienten verbessert.

Eine bevorzugte Ausführungsform der erfindungsgemäßen Testvorrichtung soll nun durch Beispiele unter Bezug auf die beiliegenden Zeichnungen beschrieben werden :

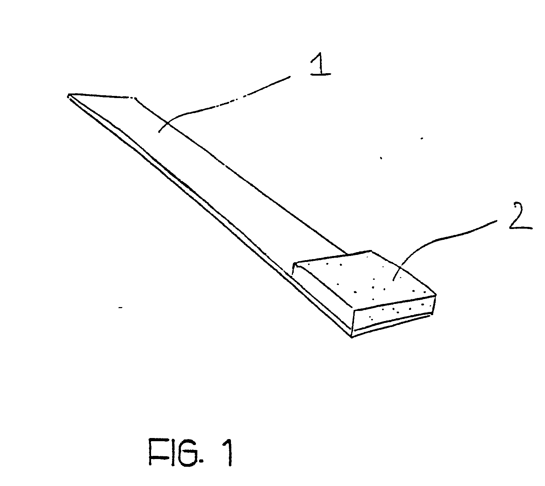

Figur 1 ist eine perspektivische Ansicht der bevorzugten Ausführungsform der erfindungsgemäßen Vorrichtung. In Figur 1 besteht die Testvorrichtung aus einem dünnen Kunststoffstreifen (1), an dessen einem Ende ein Blatt Filterpapier (2) befestigt ist, das ein Reagenz, das in der Lage ist, einen sichtbaren Farbwechsel in Gegenwart von Thiolen zu zeigen, einen Puffer und ein Anti-Oxidans ohne Thiol-Gruppe, absorbiert enthält. Bei Benutzung wird der Streifen (1) an dem, dem Blatt (2) entgegengesetzten Ende gehalten, das Blatt in eine Körperflüssigkeit wie Urin eingetaucht, herausgezogen und visuell geprüft, ob ein Farbwechsel des Reagenzes eingetreten ist oder nicht. Die Herstellung, der Gebrauch, die Sensitivität und die Stabilität der erfindungsgemäßen Testvorrichtung wird anhand der folgenden Beispiele beschrieben.

## Beispiel 1 Herstellung

Eine Reagenzlösung mit folgenden Konzentrationen wurde hergestellt :

| | |
|---|---|
| 5′,5′-Dithiobis -(2-nitrobenzoesäure) | 0,05 g |
| Trikaliumcitrat | 2,5 g |
| Chinol | 0,1 g |
| Phosphat Puffer (0,25 M, pH 7,4) ad | 25 ml |

In diese Lösung wurde Whatman No. 1 Filterpapier eingetaucht, das anschließend über Nacht langsam bei Raumtemperatur getrocknet wurde.

## Beispiel 2 Verwendung des Teststreifens

Urinproben können entweder unmittelbar nach ihrer Abgabe oder später, dann jedoch unter Zugabe von soviel EDTA, daß die Konzentration im Urin annähernd 1 mg/ml beträgt, getestet werden. Diese Maßnahme ist nötig, um die spontane Bildung von MESNA-Disulfid, das nicht detektierbar ist, zu reduzieren.

In den folgenden Beispielen wurden schmale Streifen des nach Beispiel 1 imprägnierten Filterpapiers in Urinproben getaucht. Eine leuchtendgelbe Farbe, die innerhalb weniger als 30 Sekunden erschien, wurde als Hinweis auf MESNA-haltige Urin-Proben genommen.

Zur Kontrolle wurden 8 Urinproben von 4 Männern und 4 Frauen, die kein MESNA oder keine anderen Thiole, außer den normalen, aus Speisen stammenden, erhalten hatten, durch Eintauchen der imprägnierten Testpapiere getestet. Alle zeigten keine Reaktion. Eine Reihe mit abnehmenden MESNA Konzentrationen wurde mit den nach Beispiel 1 hergestellten Testpapieren überprüft. Die Nachweisgrenze für MESNA lag bei ungefähr 0.015 mg/ml. Die Obergrenze, über der keine weitere Farbveränderung mehr beobachtet werden konnte, lag annähernd bei 2 mg/ml. Der visuell beobachtbare Farbwechsel für das imprägnierte Papier variierte von leicht blaßgelb für Konzentrationen von 0,015 bis 0,062 mg/ml über gelb für Konzentrationen von 0,025 bis 0,08 mg/ml zu orange für Konzentrationen von 0,07 - 0,6 mg/ml und höher. Die Überlappungen dieser semiquantitativen Kategorien kommen durch unterschiedliche Interpretation der Farbe durch verschiedene Personen zustande. Eine Blindprobe vor der Applikation von MESNA ist daher zu empfehlen !

## Beispiel 3 Stabilität der Teststreifen

Nach Beispiel 1 hergestellte imprägnierte Papiere wurden der normalen Laboratmosphäre im Licht für einen Monat ausgesetzt. Während dieser Zeit trat eine leichte Bräunung ein, die wahrscheinlich das Resultat der Chinol-Oxidation ist. Dieser Effekt beeinflußte jedoch nicht den Nachweis der Thiole in den Urinproben oder die Sensitivität der Technik. Nichtsdestoweniger kann die Lagerung in dunklen Behältern über Silica Gel die Verfärbungen des Papiers reduzieren.

**Patentansprüche**

1. Vorrichtung zum Nachweis von Thiolen in Körperflüssigkeiten, dadurch gekennzeichnet, daß sie ein Absorptionsmaterial enthält, in dem der Indikator 5′,5′-Dithiobis-(2-nitrobenzoesäure), ein Puffer und als Anti-Oxidans Flavonoide, Katechine, Ascorbinsäure, Chinole oder Chinolderivate absorbiert ist.

2. Vorrichtung gemäß Anspruch 1, dadurch gekennzeichnet, daß sie die Form eines Streifens, an dessen einem Ende das Absorptionsmaterial in Form einer Scheibe, eines Streifens oder eines Blattes bis zu einer Stärke von 0,5 mm gemäß Anspruch 1 befestigt ist, besitzt.

3. Vorrichtung gemäß einem der vorhergehenden Ansprüche, dadurch gekennzeichnet, daß das

4

Absorptionsmaterial Filterpapier ist.

4. Vorrichtung gemäß einem der vorhergehenden Ansprüche, dadurch gekennzeichnet, daß das Anti-Oxidans Chinol oder dessen Derivate ist.

5. Vorrichtung gemäß einem der vorhergehenden Ansprüche, dadurch gekennzeichnet, daß sie aus einem dünnen Kunststoffstreifen besteht, an dessen einem Ende ein Blatt Filterpapier, das als Indikator 5',5'-Dithiobis-(2-nitrobenzoesäure), einen Puffer und als Anti-Oxidans Chinol absorbiert enthält, befestigt ist.

6. Verfahren zum Nachweis von Thiolen in Körperflüssigkeiten, dadurch gekennzeichnet, daß eine Vorrichtung gemäß Anspruch 1 bis 5 in die zu testende Körperflüssigkeit eingetaucht wird und der Grad einer auftretenden Farbveränderung als semiquantitatives Maß für die Anwesenheit von Thiolen genommen wird.

7. Verfahren gemäß Anspruch 6, dadurch gekennzeichnet, daß der Körperflüssigkeitsprobe nach der Entnahme ein Stabilisator zugegeben wird.

8. Verfahren gemäß Anspruch 6 bis 7, dadurch gekennzeichnet, daß der Stabilisator EDTA ist, und die Konzentration des Stabilisators in der Probe ca. 1 mg/ml beträgt.


**Claims**

1. Apparatus for detecting thiols in body fluids, characterised in that it contains an absorbent material in which the indicator 5', 5'-dithiobis-(2-nitrobenzoic acid), a buffer and, as antioxidant, flavinoids, catechols, ascorbic acid, quinols or quinol derivatives are absorbed.

2. Apparatus as claimed in claim 1, characterised in that it is in the form of a strip, to one end of which is attached the absorbent material in the form of a disc, a strip or a leaf, up to a thickness of 0,5 mm as claimed in claim 1.

3. Apparatus as claimed in one of the preceding claims, characterised in that the absorbent material is filter paper.

4. Apparatus as claimed in one of the preceding claims, characterised in that the antioxidant is quinol or a derivative thereof.

5. Apparatus as claimed in one of the preceding claims, characterised in that it consists of a thin plastics strip to one end of which is attached a leaf of filter paper which contains 5', 5'-dithiobis(2-nitrobenzoic acid) as indicator, a buffer and quinol as antioxidant absorbed therein.

6. Process for detecting thiols in body fluids, characterised in that an apparatus as claimed in claims 1 to 5 is dipped in the body fluid which is being tested, and the degree of colour change occurring is taken as a semiquantitative measurement of the presence of thiols.

7. Process as claimed in claim 6, characterised in that a stabiliser is added to the sample of body fluid after it has been taken.

8. Process as claimed in claims 6 to 7, characterised in that the stabiliser is EDTA and the concentration of the stabiliser in the sample is about 1 mg/ml.


**Revendications**

1. Dispositif pour la détection de thiols dans les liquides corporels, caractérisé en ce qu'il contient un matériau d'absorption dans lequel sont absorbés l'indicateur, l'acide 5',5'-dithiobis-(2-nitrobenzoïque), un tampon, et comme antioxydant, des flavonoïdes, des catéchines, l'acide ascorbique, des quinols ou des dérivés du quinol.

2. Dispositif suivant la revendication 1, caractérisé en ce qu'il possède la forme d'une bande, à une extrémité de laquelle est fixée la matière d'absorption sous la forme d'un disque, d'une bande ou d'une feuille jusqu'à une épaisseur de 0,5 mm, conformément à la revendication 1.

3. Dispositif suivant l'une des revendications précédentes, caractérisé en ce que la matière d'absorption est du papier filtre.

4. Dispositif suivant l'une des revendications précédentes, caractérisé en ce que l'antioxydant est du quinol ou un de ses dérivés.

5. Dispositif suivant l'une des revendications précédentes, caractérisé en ce qu'il se compose d'une bande mince de matière plastique, à une extrémité de laquelle est fixée une feuille de papier filtre qui contient à l'état absorbé de l'acide 5',5'-dithiobis-(2-nitrobenzoïque) comme indicateur, un tampon et comme antioxydant, du quinol.

6. Procédé pour la détection de thiols dans des liquides corporels, caractérisé en ce qu'un dispositif suivant les revendications 1 à 5 est plongé dans le liquide corporel à essayer et en ce que le degré de la modification de couleur qui se produit est pris comme mesure semiquantitative de la présence de thiols.

7. Procédé suivant la revendication 6, caractérisé en ce qu'un stabilisant est ajouté à l'échantillon de liquide corporel après le prélèvement.

8. Procédé suivant les revendications 6 à 7, caractérisé en ce que le stabilisant est de l'EDTA et en ce que la concentration du stabilisant dans l'échantillon est d'environ 1 mg/ml.

FIG. 1